# EUROPEAN PATENT APPLICATION

(11) **EP 1 108 371 A1**
(43) Date of publication of application: **20.06.2001**
(21) Application number: 99959707.3
(22) Date of filing: 08.12.1999
(51) Int. Cl.: A41B 9/02

(54) **METHOD OF PRODUCING THROWAWAY TRUNKS TYPE WEARING ARTICLES**

(30) Priority: 09.12.1998 JP 35041498
(71) Applicant: Uni-Charm Company Limited, Kawanoe-Shi, Ehime 799-0111 (JP)
(72) Inventor: MURAKAMI, Masaki Tech. Center, Uni-Charm Co., Ltd., Mitoyo-gun Kagawa 769-1602 (JP); MATSUSHITA, Michiyo Tech. C., Uni-Charm Co., Ltd., Mitoyo-gun Kagawa 769-1602 (JP)
(74) Representative: Murgatroyd, Susan Elizabeth
(86) International application number: JP9906888
(87) International publication number: WO0033679

(57) **Abstract**

Two webs 51, 52 of same width are bonded together along peripheries of a plurality of apertures 53, 54 serially arranged in same shape and size on respective one sides of the respective webs 51, 52; these two webs 51, 52 are folded back along center lines transversely dividing the respective webs 51, 52 onto themselves; opposite side edges 57, 57 as well as 60, 60 of these respective two webs 51, 52 are bonded together; and finally these two webs 51, 52 thus bonded together are cut along first and second imaginary lines G-G, H-H extending transversely of the webs 51, 52.

## Description

### FIELD OF THE INVENTION

This invention relates to a process for making disposable trunks-type garment.

### BACKGROUND ART

Japanese Patent Application Disclosure Gazette No. Hei3-111047 discloses a process for making a disposable garment. This process enables a briefs-type garment to be continuously made. According to the process disclosed in this document, a web assembly including a liquid-absorbent core and provided in its transversely middle zone with a series of apertures is continuously fed in one direction and folded along a center line dividing a width of this web assembly in two back onto itself. Two sections of the web assembly folded back onto itself in this manner are then bonded together in an intermediate region defined between each pair of adjacent apertures. The web assembly folded back onto itself and bonded together is finally cut along a line extending orthogonally to the center line so as to divide each aperture in two and along a line also extending orthogonally to the center line so as to divide a section defined between each pair of adjacent apertures in two. In this manner, individual briefs-type garments are obtained. The garment obtained by this process has its component webs put flat and bonded together along transversely opposite side edges of its trunk region.

The briefs-type garment of the prior art is disadvantageous so far as its external appearance is concerned because the component webs put flat and bonded together along the transversely opposite side edges of the trunk region inevitably results in an emphasized width of a wearer's waist zone as viewed from the front of the wearer. Some of the consumers may dislike such garment. In addition, the process disclosed in the document is suitable for making the briefs-type garment but unsuitable for making the trunks-type garment.

In view of the problem as has been described above, it is a principal object of this invention to provide a simple and easy process for making a disposable trunks-type garment adapted to avoid the weakness that the width of the wearer's waist zone might be emphasized.

### SUMMARY OF THE INVENTION

The object set forth above is achieved, according to this invention, by a process for making a disposable trunks-type garment from two sheets of a same width placed one upon another and continuously fed in one direction, the process at least comprising the steps of:
a. bonding the two sheets, each having a plurality of apertures arranged at given intervals on one side of and in parallel to a center line transversely dividing the sheet, the apertures being identical in shape as well as in size to those arranged on one side of and in parallel to a center line transversely dividing the other sheet, the two sheets being placed one upon another so that the apertures of the two sheets are exactly in alignment, to each other along peripheries of these aligned apertures and continuously feeding these two sheets bonded together in this manner;
b. folding the sheets back onto themselves along the center lines, respectively, so that opposite side edges of the respective sheets are placed one upon another exactly in alignment;
c. bonding together the opposite side edges of the sheets placed one upon another, respectively;
d. after the step c), cutting the two sheets along along a first cutting line extending orthogonally to the center lines so as to divide the aperture in two; and
e. after the step c), cutting the two sheets along a second cutting line extending orthogonally to the center lines so as to divide a section of the sheets extending between each pair of adjacent said apertures.

According to one preferred embodiment of this invention, the process further comprises, before the step d), a step of bonding elastic members with a tension to the two sheets so that the elastic members extend in parallel to the first cutting line on both sides of the first cutting line.

According to another preferred embodiment of this invention, the two sheets are placed one upon another with their center lines being exactly in coincidence.

According to still another preferred embodiment of this invention, the two sheets are placed one upon another with their center lines being spaced from each other by 1/2 width of the sheets.

### BRIEF DESCRIPTION OF THE DRAWINGS

Fig. 1 is a perspective view showing a garment (shorts) constructed according to a principle of this invention as partially broken away;
Fig. 2 is a schematic diagram illustrating the steps of a process for making the garment (shorts);
Fig. 3 is a schematic diagram illustrating a step of the process according to one preferred embodiment of this invention;
Fig. 4 is a perspective view of the shorts obtained through the steps of the process illustrated by Fig. 3;
Fig. 5 is a schematic diagram illustrating a step of the process according to another preferred embodiment of this invention; and
Fig. 6 is a perspective view of the shorts obtained through the steps of the process illustrated by Fig. 5.

### DESCRIPTION OF THE BEST MODE FOR WORKING OF THE INVENTION

Details of a process according to this invention for making a disposable trunks-type garment will be more fully understood from the description given hereunder in reference with the accompanying drawings.

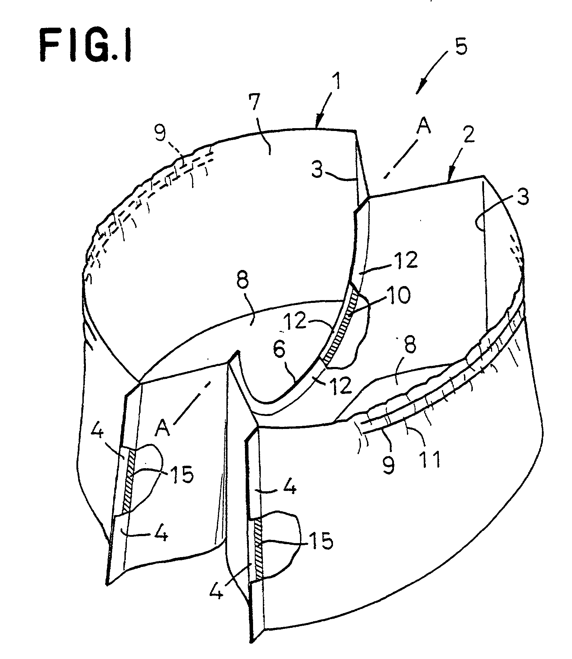

Fig. 1 shows shorts 5 as a specific example of a disposable trunks-type garment made by a process according to this invention in a perspective view as partially broken away, in which the left-hand of the figure corresponds to the front side of the shorts 5. The shorts 5 have a right-half lateral body 1 destined to cover a right-half of a wearer's trunk and a left-half lateral body 2 destined to cover a left-half of the wearer's trunk. These two bodies 1, 2 are respectively formed by single sheets and symmetric to each other with respect to a vertical plane including a center line A-A dividing a trunk region of the shorts 1 in right- and left-halves. Taking account of such symmetric relationship, corresponding regions in the right- and left-half bodies 1, 2 will be designated by similar reference numerals in the following description unless any particular notice is given. Each of the right- and left-half lateral bodies 1, 2 has a folding line 3 vertically extending on the rear side of the shorts 5 and sheet's opposite side edges 4 vertically extending on the belly side of the shorts 5. The opposite side edges 4 are put flat together and bonded to each other along a line 15. The right- and left-half lateral bodies 1, 2 have substantially U-shaped cutouts 12 destined to form together a transition of a crotch region 6 between belly and back sides of the shorts 5. The right- and left-half lateral bodies 1, 2 are bonded to each other along a line 10 defined by peripheral edges of the respective cutouts 12. The right- and left-half lateral bodies 1, 2 bonded together in this manner form a waist-opening 7 and a pair of leg-openings 8 adapted to surround the wearer's thighs at a level lower than the crotch region 6. Lateral regions of the respective half bodies 1, 2 are provided on outer or inner surfaces thereof with a plurality of elastic members 9 bonded with a tension to the respective half bodies 1, 2. Contraction of these elastic members 9 generates a plurality of gathers 11 on both lateral regions of the shorts 5.

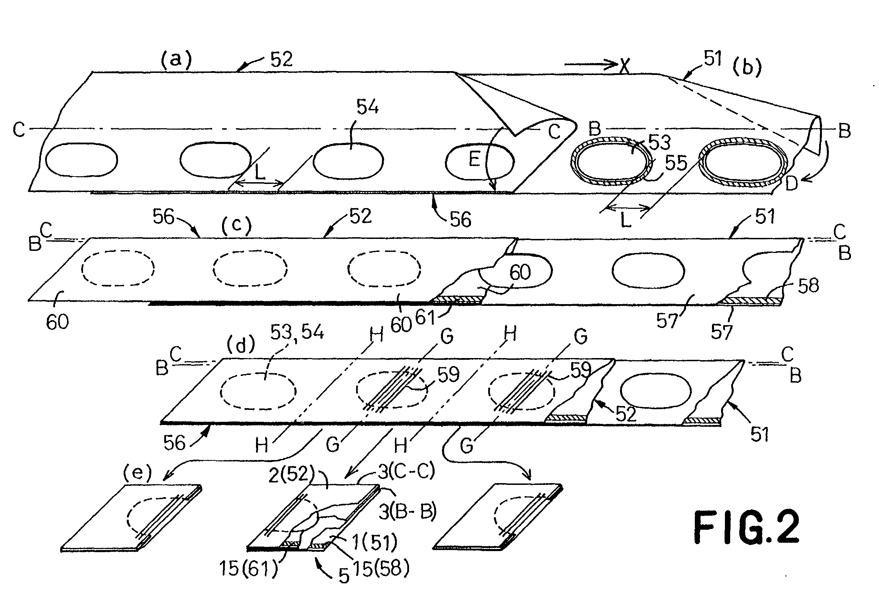

Fig. 2 is a schematic diagram illustrating the steps of a process for making the shorts 5 shown by Fig. 1. On a step a), first web 51 destined to form the right-half lateral body 1 and second web 52 destined to form the left-half lateral body 2 are continuously fed in a direction indicated by an arrow X. The first and second webs 51, 52 have substantially same width and are placed upon each other so that center lines B-B, C-C transversely dividing the webs 51, 52 in two, respectively, coincide with each other. The first and second webs 51, 52 have on respective one sides of the center lines B-B, C-C a plurality of first and second apertures 53, 54 which are identical in shape as well as in size and arranged at desired intervals L along the center lines B-B, C-C. These two webs 51, 52 are placed upon each other with the first and second apertures 53, 54 being exactly in alignment and bonded to each other along lines 55 defined by peripheries of the respective apertures 53, 54. In this manner, a laminated sheet 56 is obtained.

On a step b), the first web 51 is folded back along the center line B-B in a direction indicated by an arrow D onto itself and, similarly, the second web 52 is folded back along the center line C-C in a direction indicated by an arrow E onto itself.

On a step c), opposite side edges 57 of the first web 51 folded back onto itself are bonded together along a line 58 and, similarly, opposite side edges 60 of the second web 52 folded back onto itself are bonded together along a line 61.

On a step d), sections of the first and second webs 51, 52 folded back on the precedent step c) are respectively provided on their outer surfaces with elastic members 59 bonded with a tension thereto so that these elastic members 59 extend on both sides of each imaginary line G-G bisecting each diameter of the aperture 53, 54 transversely of these webs 51, 52.

On a step e), the laminated sheet 56 is cut along the first imaginary line G-G and along a second imaginary line H-H (See Fig. 2d)) extending orthogonally to the center lines B-B, C-C so as to divide a section extending between each pair of adjacent apertures 53, 54. In this manner, the individual shorts 5 are obtained.

The center lines B-B, C-C of the first and second webs 51, 52 used during the steps of the process as have been described above are destined to form the folding lines 3 of the shorts 5 shown by Fig. 1. The opposite side edges 57, 60 of the respective webs 51, 52 and bonding lines 58, 61 are destined to form the sheets' side edges 4 and the bonding lines 15 of the shorts 5, respectively. The first and second apertures 53, 54 divided in two are destined to form the U-shaped cutouts 12 of the crotch region 6 and the bonding lines 55 extending along the peripheries of the respective apertures 53, 54 are destined to form the bonding line 10 of the crotch region 6.

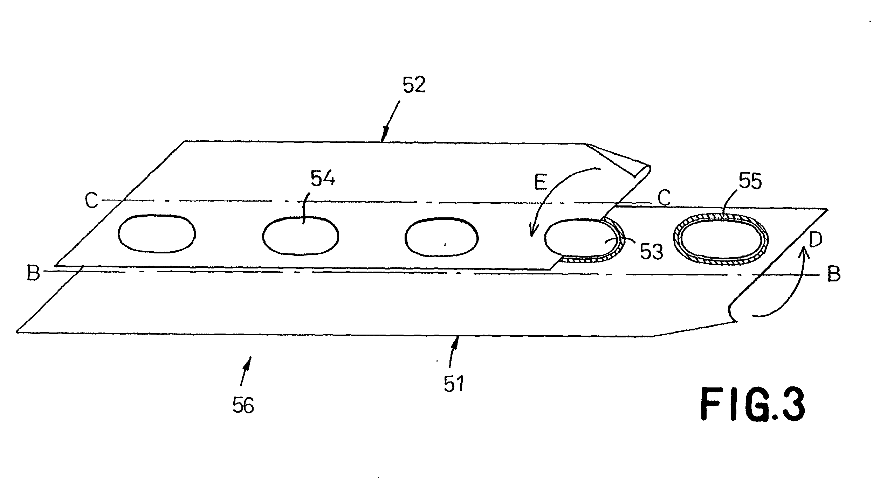

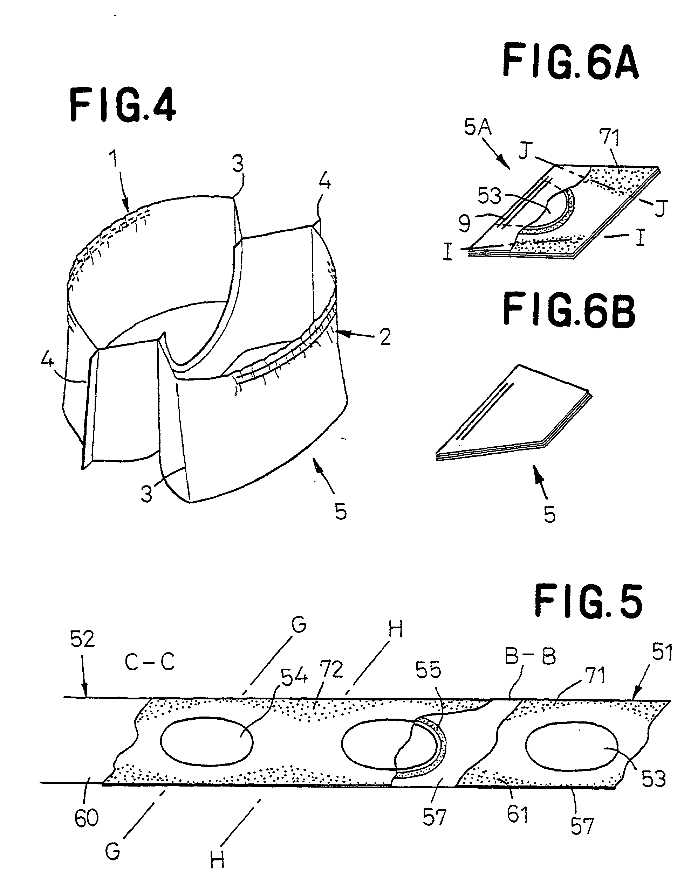

Fig. 3 is a schematic diagram similar to Fig. 2a) illustrating a step of the process according another preferred embodiment of this invention and Fig. 4 is a perspective view of shorts 5 obtained by the process illustrated by Fig. 3. Referring to Fig. 3, the first web 51 and the second web 52 are transversely shift relative to each other by 1/2 width of the web so as to overlap each other. This overlapping sections are formed with the first and second apertures 53, 54. The first and second webs 51, 52 are bonded together along the lines 55 defined by the peripheries of the respective apertures 53, 54. The first web 51 is folded back in a direction indicated by an arrow D and the second web 52 is folded back in a direction indicated by an arrow E. In the case of the shorts 5 obtained by the process according to this embodiment, the folding line 3 of the right-half body 1 formed by the first web 51 lies on the back side and the sheet's opposite side edges 4 lie on the belly side of the wearer. The folding line 3 of the left-half body 2 formed by the second web 52 lies on the belly side and the sheet's opposite side edges 4 lie on the back side of the wearer.

Fig. 5 is a schematic diagram illustrating a step of the process according to another preferred embodiment of this invention and Fig. 6 is a perspective view of the shorts 5 obtained by the process illustrated in Fig. 5. Referring to Fig. 5, the first and second webs 51, 52 are bonded together along the bonding lines 55 defined by the peripheries of the respective apertures 53, 54. The first web 51 is folded back along the center line B-B onto and bonded to itself over a relatively large area indicated by a plurality of dots 71 and the second web 52 is bonded to itself over a relatively large area indicated by a plurality of dots 72. Fig. 6A is a perspective view showing the shorts 5A obtained by cutting the first and second webs 51, 52 bonded together in this manner along the first and second imaginary lines G-G, H-H as partially broken away. Since the first and second webs 51, 52 are bonded to themselves over the relatively large areas, respectively, the shorts 5A may be trimmed along third and fourth imaginary lines I-I, J-J a distance between which progressively reduces from the waist-opening toward the leg-openings to obtain the shorts 5 shown by Fig. 6B. The shorts 5 of this arrangement enables. a circumferential dimension to be varied from the waist-opening toward the leg-openings.

To implement this invention, sheets made of appropriate materials such as a nonwoven fabric, a woven fabric and a plastic film may be used as stock materials for the first and second webs 51, 52. These webs 51, 52 may be bonded together by use of an adhesive agent such as hot melt adhesive or a sealing technique such as heat- or ultrasonic-sealing technique. Concerning the elastic members 59 provided on the both lateral sides of the shorts 5, it is possible to bond these elastic members 59 to the lower surface of the first web 51 as well as to the upper surface of the second web 52, instead of following the step a) in Fig. 2. If the first and second webs 51, 52 themselves have an adequate elasticity in the transverse direction, the elastic members 9, 59 serving to give the shorts 5 the circumferential elasticity may be eliminated. Furthermore, if the first and second webs 1, 2 are made of thermoplastic stock material, the folding lines 3 will not easily disappear once the thermoplastic stock material has been folded under a heating along the center lines B-B, C-C and a shape stability of the shorts 5 will be improved.

The shorts 5 obtained by the process according to this invention may be used not only as disposable undergarment but also as disposable upper garment. Furthermore, an appropriate body fluids absorbent member may be provided on the inner side thereof to use the shorts 5 also as disposable trunks-type diaper. In this manner, the shorts 5 according to this invention can be used as a wide variety of disposable trunks-type garments.

The process according to this invention enables disposable trunks-type garment to be continuously made. The garment obtained by this process is characterized by that the sheets as the stock material for the shorts are not bonded together at both lateral sides of the wearer's trunk and, therefore, the waist zone of the shorts never appears uselessly enlarged when the shorts are actually put on a wearer's body. The external appearance of the shorts is significantly improved particularly as viewed from the front of the wearer.

## Claims

1. A process for making a disposable trunks-type garment from two sheets of a same width placed one upon another and continuously fed in one direction, said process at least comprising steps of:
a. bonding said two sheets, each having a plurality of apertures arranged at given intervals on one side of and in parallel to a center line transversely dividing this sheet, said apertures being identical in shape as well as in size to those arranged on one side of and in parallel to a center line transversely dividing the other sheet, said two sheets being placed one upon another so that said apertures of said two sheets are exactly in alignment, to each other along peripheries of these aligned apertures and continuously feeding these two sheets bonded together in this manner;
b. folding said sheets back onto themselves along said center lines, respectively, so that opposite side edges of the respective sheets are placed one upon another exactly in alignment;
c. bonding together said opposite side edges of said sheets placed one upon another, respectively;
d. after the step c), cutting said two sheets along a first cutting line extending orthogonally to said center lines so as to divide said aperture in two; and
e. after the step c), cutting said two sheets along a second cutting line extending orthogonally to said center lines so as to divide a section of said sheets extending between each pair of adjacent said apertures.

2. A process according to Claim 1, further comprising, before said step d), a step of bonding elastic members with a tension to said two sheets so that said elastic members extend in parallel to said first cutting line on both sides of said first cutting line.

3. A process according to Claim 1 or 2, wherein said two sheets are placed one upon another with their center lines being exactly in coincidence.

4. A process according to Claim 1 or 2, wherein said two sheets are placed one upon another with their center lines being spaced from each other by 1/2 width of the sheets.
